# EUROPEAN PATENT APPLICATION

(11) **EP 2 375 245 A1**
(43) Date of publication of application: **12.10.2011**
(21) Application number: 11172538.8
(22) Date of filing: 27.10.2009
(51) Int. Cl.: G01N 27/30, G01N 33/18

(54) **Capacitance electrode and sensor-system capable of sensing contaminants and method therefor**

(30) Priority: 27.10.2008 NL 1036116; 03.03.2009 NL 1036657
(62) Divisional of application: 09751987.0
(71) Applicant: Smart Frequencies B.V., 8501 XH Joure (NL)
(72) Inventor: Mayer, Mateo Jozef Jacques, 8934 CJ Leeuwarden (NL); De Smet, Louis Cornelia Patrick Maria, 8934 CJ Leeuwarden (NL); Sudhölter, Ernst Jan Robert, 8934 CJ Leeuwarden (NL)
(74) Representative: Verdijck, Gerardus

(57) **Abstract**

The invention relates to an electrode, and a sensor-system for capacitively and/or photovoltaic sensing contaminants in a fluid. The invention also relates to a micro-reactor comprising such sensor-system and methods for measuring and producing. The capacitance electrode comprises:
- a transducing element (6);
- an affinity layer (10) that in use is in contact with a fluid to be measured, wherein the affinity layer comprises a multi-array surface; and
- an organic monolayer insulating layer (8) separating the transducing element and the affinity layer,
wherein the electrode is arranged such that in use contaminants in the fluid may interact with the affinity layer.

## Description

The present invention relates to a capacitance electrode for use in a sensor-system. The electrode is capable of capacitively sensing contaminants in a fluid. An example of a fluid is water and the electrode can for example be used in the production of high-quality drinking water and industrial water.

Existing sensing techniques include the determination of the binding of a contamination and a sensor surface using Surface Plasman Resonance (SPR) and Mach-Zehnder Interferometry. Most sensor-systems that are used in practice measure a specific contaminant in a fluid like water. For example, the presence of nitrite anions in water can be measured using W-spectra on a surface of the sensor that changes in response to bonding of the nitrite anions to the specific surface layer. Other sensing techniques to measure the presence of specific contaminants include fluorescence and radiochemical labelling, impedance measurements,

In order to exclude all safety risks related to ending up of these contaminations in for example drinking water it would be required to analyse the water on an unacceptably high number of contaminations using the existing sensing techniques. As an illustration, the current Water Framework Directive comprises a list of 33 toxic components with an EU-Water Quality specification Directive. This list is about to be extended with more than 100 additional components that will possibly be included in the water specification in near future. Furthermore, the number of potentially toxic components that may end up in the drinking water, like pharmaceuticals, is continuously increasing. The concentration of all these components will gradually increase in a complete water chain to a steady concentration at which the total amount of toxic components removed through purification equals the amount of toxic components introduced into the water chain. Because of this large number of toxic components and a usually very low concentration in water, the detection, of these components in for example surface water or drinking water is expensive, labour intensive and off-line, Another problem relates to determining a concentration of contaminants instead of determining the mere presence of a contamination.

The present has for its object to provide a capacitance electrode usable in a sensor-system that is capable of capacitively sensing contaminants in a fluid that is more efficient than existing devices and methods.

This objective is achieved with the electrode according to the invention, the electrode comprising:
- a transducing element;
- an affinity layer that in use is in contact with a fluid to be measured, wherein the affinity layer comprises a multi-array surface; and
- an insulating layer separating the transducing element and the affinity layer,
wherein the electrode is arranged such that in use contaminants in the fluid may interact with the affinity layer,

The basis for the electrode is defined by a transducing element or transducing layer. At least one surface of this transducing element is provided with a skinny affinity layer. The detection principle is based on the interaction of the contaminants with this affinity layer. Contaminations include dissolved components, suspended particles and micro-organisms. This may include organic and inorganic compounds, such as pharmaceuticals. The transducing element and the affinity layer are separated by an insulating layer. The transducing element can be made of a silicon substrate. On at least one side of this silicon substrate the insulating and affinity layers are provided. Preferably on the back side of this silicon substrate electrical contacts are provided, The set-up of the sensor-system in which the capacitance electrode is used is that of an electrical capacitor in which the two parallel capacitor plates are the silicon substrate and the skinny affinity layer.

A thin insulating layer (of thickness d) contributes to a high capacity C, since C= (A × ε₀)/d, where ε_{D} is the permittivity of the organic monolayer, and A is the surface area of the plate. Interaction between the skinny affinity layer and contaminants in the sample solution results in a change of charge AQ at the affinity layer, which is mirrored in the silicon substrate as a change in charge of -ΔQ. That change of charge or change of charge density, ΔO = ΔQ/A, changes the electrical field by ΔE= Δσ/ε₀ which gives rise to a change of electrical potential ΔV = (ΔQ × d) / (A × ε₀) = ΔQ/C. This chemical sensor is a label-free sensor with electrical read out.

The electric properties of the affinity layer can be very sensitive to contaminations that are present in the fluid. The contaminations interact with the affinity layer, thereby changing the electric properties as mentioned above. The measure of the change in the electric properties is a measure for the type of contaminant in the fluid. Furthermore, the measurement can give a measure for the concentration of this contamination.

The affinity layer can be realised using physisorption and chemisorption. Examples are molecular imprinted polymers, emulsion polymerisation, solvent casting.

Preferably, the multi.-array surface of the affinity layer is a 2x2, 4x4, 8x8 or 16x16 multi-array. One possible way of producing the arrays is by photolithography. Preferably the different spots in the array have a diameter between 50 µm to 1 mm. The spots can be made of different materials with each material having its own characteristic behaviour. The electrode according to the present invention is a multi-array, label-free chemical electrode for the detection of contaminants in a fluid, The different materials have different polarities and affinities. These different properties influence the extent of interactions of a specific spot with the contaminants that are present in the fluid. From interactions with one spot or interactions with a combination of different non-selective spots a measure for the presence of a contaminant in the fluid is achieved. This is an indication of the mere presence of a contaminant. In addition, the measure also gives an indication on the concentration of the contaminant. The combination of different non-selective interactions forms a type of fingerprint response of the multi-array sensor.

The multi-array surface preferably comprises different polymers and polymer mixtures. The different are provided on the spots of the multi-array surface layer. It is possible to use for example accurel (a micro-poreus polypropylene membrane) wherein the pores are filled with specific fluids with characteristic electrical properties. Examples of different polymers and/or polymeric mixtures are the use of cyclo-dextrine modified polymers and plasticizers. The change of the membrane capacity due to the partition equilibrium between the fluid and the membrane can be measured.

In a preferred embodiment according to the present invention the insulating layer comprises an organic mono-layer,

Known electrodes use a. SiO₂ insulating layer. The use of an organic monolayer has the advantage that a Si-C bound mono-layer is more stable, Furthermore, cations that diffuse into the SiO₂ layer, may cause noise in the measurement. By using organic mono-layers this noise is prevented. In addition, organic mono-layers show better insulating properties compared to **SiO₂** layers of an equal thickness.

In a preferred embodiment according to the present invention the insulating layer comprises an alkene and/or alkyne that is provided with a functionalised component.

An alkene and/or alkyne of the insulating layer can connect to the transducing layer with Si - H. The alkene and/or alkyne can be functionalised to provide the desired properties for the specific circumstances of the application, Functionalising the mono-layer can be realised directly using a functional group that does not react with Si-H. Also, an indirect approach is possible using a functional group that does not react with Si-H. In a further step the desired molecule is connected. Optionally, this indirect approach can be performed using a functional protected group that is combined with the desired molecule after obviating the protection. Examples of functional groups are COOH and NH**₂,** Other examples are OH and CN. As OH-COOH- and NH₂-groups may react with Si-H these groups are, in an indirect approach, protected using esters and amides, for example.

In a preferred, embodiment according to the present invention the affinity layer substantially comprises particles smaller than 1 mm, such that the layer substantially acts as a conductor when exposed to a high-frequent alternating current and acts as an insulator when exposed to a direct current.

By providing the relatively small particles with a diameter below 1 mm it is possible to manipulate the adsorption characteristic. This adsorption characteristic may be manipulated when applying for example an alternating current between electrodes or between a working electrode and a counter electrode. The manipulation of the adsorption characteristic may improve the sensitivity of a sensor using the electrode according to the present invention.

The invention further relates to a sensor-system for measuring contaminants in a fluid, comprising:
- a working electrode comprising a capacitance electrode as described above;
- a counter electrode arranged such that in use fluid to be measured separates the working and counter electrode; and
- a potentiostat capacitively sensing the change in electric properties caused by adsorption of contaminants to the affinity layer.

With the sensor-system comprising a working electrode as mentioned above, the same effects and advantages as those stated to the electrode are provided by the sensor-system. The system uses the specific adsorption of contaminants on the functionalised electrode surface that is part of a condensator in the fluid to be measured. This functionalised electrode is the working electrode of the sensor-system. It is possible to provide more than one working electrode in the sensor-system according to the present invention. A counter electrode is also provided in the fluid. A reference electrode can be used optionally. The counter electrode comprises a conductive material, like a metal, carbon, active carbon, with a polymer layer on this conductive layer. Preferably, the electrode surface is relatively large, for example above 0.1 mm². Properties of the surface can be characterised using for example impedance spectroscopy. In the array of spots in the affinity layer, every spot has its own specific sensitivity to specific contaminants in the fluid. As mentioned above, both individual measurements and a combination of measurements provide information on the type of contaminants and the concentration thereof in the fluid. This type of measurement can be considered as a fingerprint response measurement of the multi-array sensor-system. Such fingerprint sensor can for example be used to track changes of the water quality to detect an event, like an illegal purge, an accident, heavy rainfall etc. Based upon the measurements quick measures can be taken, such as stopping the intake of water or changing to a different water source. If required additional measurements can be performed to determine more accurately concentration values by measuring offline, using the fingerprint response as a guide. Especially water fingerprinting sensor-systems that provide real-time information and that are sensitive to a specific group of organic/ inorganic compounds, such as pharmaceuticals, will contribute to safeguarding the quality of clean water.

In a preferred embodiment according to the present invention, the affinity layer of the working electrode is arranged in a cylindrical tube.

The multi-array surface of the affinity layer can be considered as a two-dimensional surface area. By providing the surface area in for example a cylindrical tube, through which a fluid is being transported, a three-dimensional surface area is constructed. By studying the measurements and especially the change measurements in time additional information is measured. Within the tube a type of chromatography process takes place and the adsorption characteristic of each contaminant in each of the spots in the multi-array surface gives information on the variation in residence time of this contaminant. This involves not only static measurements, but also involves more dynamic measurements. Off course, depending on the dimensions of the two-dimensional surface area, this also applies for this two-dimensional array.

Preferably, flow means are provided to expose the sensor in use to a continuous flow of fluid. This measurement can be performed online. Depending on the dimensions an at-line solution is also possible according to the invention. In this at-line solution, part of the fluid in controller quantities is transported along the surface area of the sensor. In case of a tube-like configuration, performing the dynamic measurements leads to a sort of four-dimensional measurement.

In a further preferred embodiment according to the present invention, the sensor-system comprises electrical means for providing a potential difference between the working- and counter electrode.

Applying a potential difference between the working electrode and the counter electrode of the sensor-system, manipulates the adsorption characteristics. This changes the sensitivity of the sensor-system, such that the sensor-system can be adapted towards the specific needs like measuring a specific class of contaminants in drinking water for example.

Preferably, the sensor-system further comprises additional electrical means for providing an alternating electrical field between the working and counter electrodes.

Providing an alternating electric field between the electrodes of the sensor-system manipulates the adsorption of contaminants in the fluid. Especially the adsorption characteristics of hydrophilic components and ions can be manipulated. This can be used to improve the sensitivity of the sensor-system in a specific range.

In a further preferred embodiment according to the present invention the counter electrode comprises an affinity layer.

By also providing the counter electrode with a functionalised layer, more specific measurements can be performed. The properties of the affinity layers on both the working- and counter electrodes can be equal or different depending on the specific application for the sensor-system.

The invention further relates to a sensor-system for detecting contaminations in a fluid and/or for treating a fluid, the system comprising:
- a working element comprising an affinity layer that in use is in contact with a fluid to be measured, and wherein the working element is arranged such that in use contaminants in the fluid may interact with the affinity layer; and
- photovoltaic means.

Such sensor-system provides the same effects and advantages as those stated with reference to the electrode and the described above. Using the affinity layer as described above in several embodiment for the capacitance electrode and sensor-system a type of photovoltaic sensor-system is achieved. Such photovoltaic sensor-system enables detecting contaminants in a fluid and/or treating a fluid, making use of photovoltaic means, a functionalized surface shaped as an affinity layer, which preferably forms part of the photovoltaic means, or is arranged thereon, and to which adsorption of the components present in the fluid can take place, at least a source which provides the photovoltaic means with electromagnetic waves, including light, and means for directly or indirectly measuring and/or directly or indirectly utilizing the current produced by the photovoltaic for electrolysis in and/or disinfection of the fluid. The technology according to the present invention is suitable as sensor for detecting contaminants in water, for the purpose of disinfecting water and for directly or indirectly measuring and influencing the metabolism of in general, and algae and chemoluminescent and/or bioluminescent bacteria in particular.

In the field of water process technology guaranteeing the reliability of delivery and the water quality of drinking water are of particular importance. Owing to the range of toxic components and organisms which may be present in surface water and spring water, rapid analysis of the water quality represents a challenge. A cost-effective method of monitoring the quality of water is to apply "fingerprinting sensors", Such a sensor detects changes in the water quality with relatively simple means and online. If a change is detected, there is then an offline investigation into what type of contaminant(s) is involved and a decision is made as to whether or not to intervene in the process. It is noted that a fingerprinting sensor is preferably based on physical measurement principles, so that no chemicals need be dosed and maintenance of the sensor is limited. It is further noted that the fingerprinting sensor preferably has some selectivity and capacity to distinguish different groups of toxic components present in the water. This latter makes it possible, in the case that the fingerprinting sensor generates an alarm, to already gain an indication of group of contaminants it is necessary to look for offline. Finally, it is noted that an array of fingerprinting sensors, each with a specific sensitivity to a group of toxic components, is a particularly cost-effective aid for guaranteeing the quality of water.

The sensor-system according to the present invention enables an effective online sensor which preferably makes use of only physical principle and is preferably applied in array form_{,} wherein each element of the sensor array has a specific sensitivity to one or more groups of toxic components in the water. As will be set fourth hereinbelow in this application, the sensor is not only suitable for detecting toxic components in water, but can be widely utilized in the chemical process industry, the pharmaceutical industry, the food industry, in biotechnological processes and in medical applications,

According to a first aspect, the technology consists of photovoltaic means. This its preferably a classical photovoltaic cell. (PV cell) consisting of a piece of semiconductor material having a separating surface therein between a p-type and an n-type doping.

According to a second aspect, the present invention consists of a preferably functionalized surface situated in a fluid so that contaminants present in the water adsorb specifically to this surface. The functionalized surface is preferably arranged on the part of the photovoltaic cell which is designed to receive and convert electromagnetic radiation to electrical energy. It is however also possible to place the surface which adsorbs the contaminants in the fluid and the PV cell outside the fluid, wherein the electromagnetic radiation which can be received by the PV cell is guided first through the functionalized surface,

According to a third aspect, the present invention consists of at least an electromagnetic transmitter which preferably emits light which is first guided through the functionalized surface before reaching the PV cell. It is noted that can serve as electromagnetic transmitter, as well as LEDs including RGB LEDs, UV LEDs, infrared LEDs, electroluminescent foil (EL foil), gas discharge tubes including UV lamps, neon lamps and sodium lamps. It will be apparent to the skilled person that each of these radiation sources has a specific frequency spectrum which, in combination with the present invention, results in a specific sensitivity to different types of contaminants in the fluid. RGB Lends are particularly interesting in combination, with the present invention because they are small, waterproof and inexpensive and can be controlled as light source located in a single point and having an adjustable frequency spectrum. It is further noted that not only light, but also other electromagnetic waves, including radio waves, can be applied in combination with the present invention. It is finally noted that the selectivity and sensitivity of a sensor according to the present invention can be increased by applying modulated electromagnetic waves.

According to a fourth aspect, the present invention consists of means for measuring the electric power generated by the PV for instance by connecting an electrical load to the PV cell and measuring a current through the cell and/or the voltage over the PV cell. It will be apparent to the skilled person that not only the absolute power measured in the stationary situation by the PV cell comprises interesting information concerning components present in the liquid, so too does the dynamic progression of the measured electrical signal and the progression of the amplitude versus frequency characteristic for different types of input signal of the electromagnetic transmitter. A non-limitative list of input signals which are particularly suitable for application, in combination with the present invention is monochromatic light, modulated light including amplitude-modulated light, pulsed light, light with a wide frequency spectrum in the infrared region, the visible region, the UV region or combinations of one or more of the above stated types of light, radio waves including amplitude-modulated and/or frequency-modulated and/or sideband-modulated and/or phase-modulated waves.

The invention further relates to a micro-reactor comprising a sensor-system as described above.

The micro-reactor provides the same effects and advantages as those stated with reference to the sensor-system for capacitive and/or photovoltaic sensing. More specific, the micro-reactor is capable to decompose components that adsorb to the functionalised surface area of the electrode or working element. In preferred embodiments for the micro-reactor according to the present invention the surface area of the affinity layer is somewhat smaller than the electrode or working element. Furthermore, preferably a polymer is used that acts as an insulator for direct currents and as a conductor of alternating currents.

The invention further relates to a method for measuring contaminants in a fluid, the method comprising the steps of:
- providing a sensor-system as described above; and
- capacitive and/or photovoltaic sensing the change in electric properties of the affinity layer due to adsorption of contaminants of the affinity layer.

Such method provides the same effects and advantages as those stated with reference to the electrode and sensor-system.

The invention further also relates to a method for producing a capacitance electrode, and/or working element, comprising an affinity layer as mentioned above, the method comprising the steps or
- etching a silicon surface;
- hydrosilylating the silicon surface to form a linked organic monolayer; and
- depositing the affinity layer.

Such method provides the same effects and advantages as those stated with reference to the electrode and the sensor-system. Preferably, the depositing of the affinity layer involves different polymers or polymeric mixtures to form a multi-array affinity layer.

Further advantages, features and details of the invention are elucidated on a basis of preferred embodiments thereof, wherein reference is made to the accompanying drawings in which:
- figure 1 shows an overview of the sensor-system according to the invention;
- figure 2 illustrates the method to produce the electrode according to the invention;
- figure 3A+B illustrate spots of the multi-array surface;
- figure 4 illustrates the method to produce the surface of figure 3;

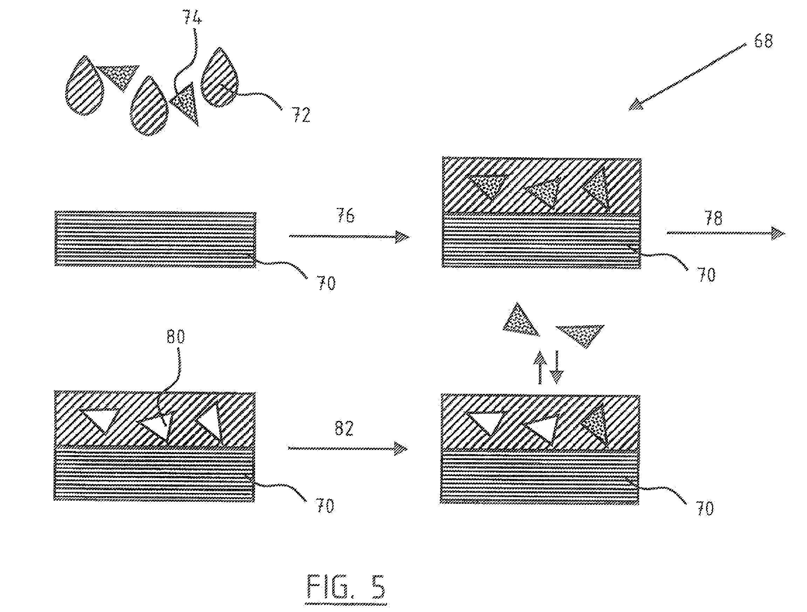

- figure 5 illustrates the method to include particles in the functionalised surface layer; and

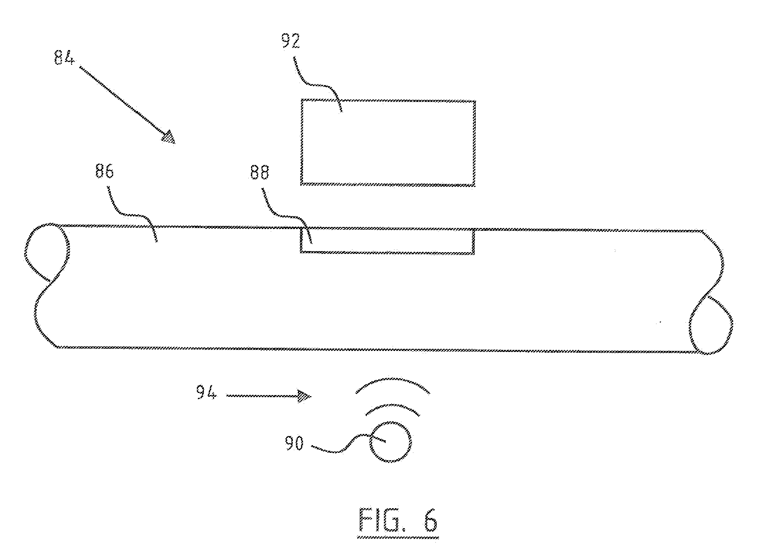

- Figure 6 illustrates a sensor-system making use of photovoltaic means,

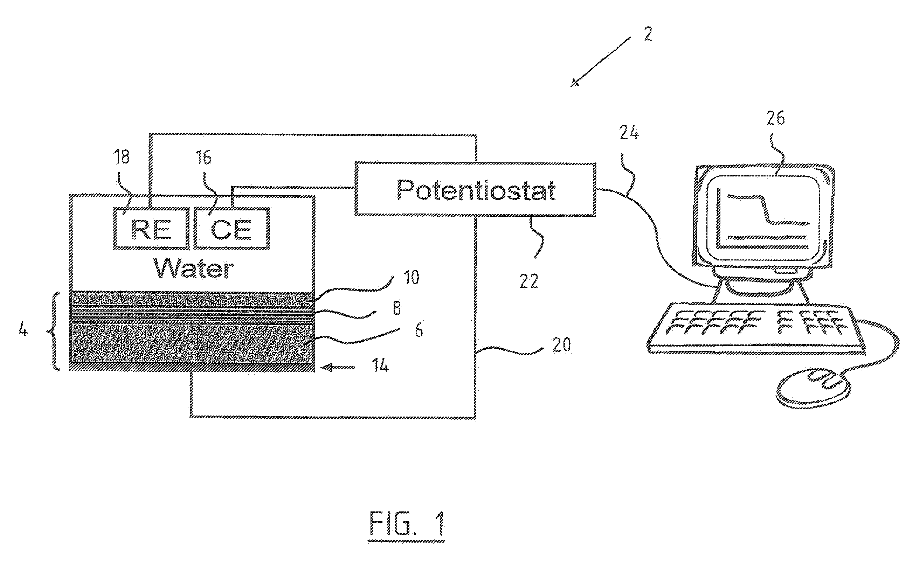

A sensing system 2 (figure 1) comprises working electrode 4. Working electrode 4 comprises a base layer 6 of a silicon substrate. Substrate 6 is covered with an organic monolayer 8 acting as a thin nano-sized insulator between the substrate 6 and the skinny affinity layer 10. The affinity layer 10 is in contact with the fluid 12. One side of substrate 6 is provided with the mono-layer 8 and affinity layer 10. The other side of substrate 6 is provided with a back contact 14. Sensing system 2 further comprises a counter electrode 16 and a reference electrode 18. The counter electrode 16 is for example made of platinum and the reference electrode is for example made from AgCl/Cl. These electrodes 16, 18 are in the fluid 12. The electrodes 4, 16, 18 are connected through circuit 20 to a Potentiostat 22. Potentiostat 22 is connected by connection or circuit 24 to a computer 26. Computer 26 is used for control of the sensing system 2 and is also be used for monitoring the measurements performed by system 2.

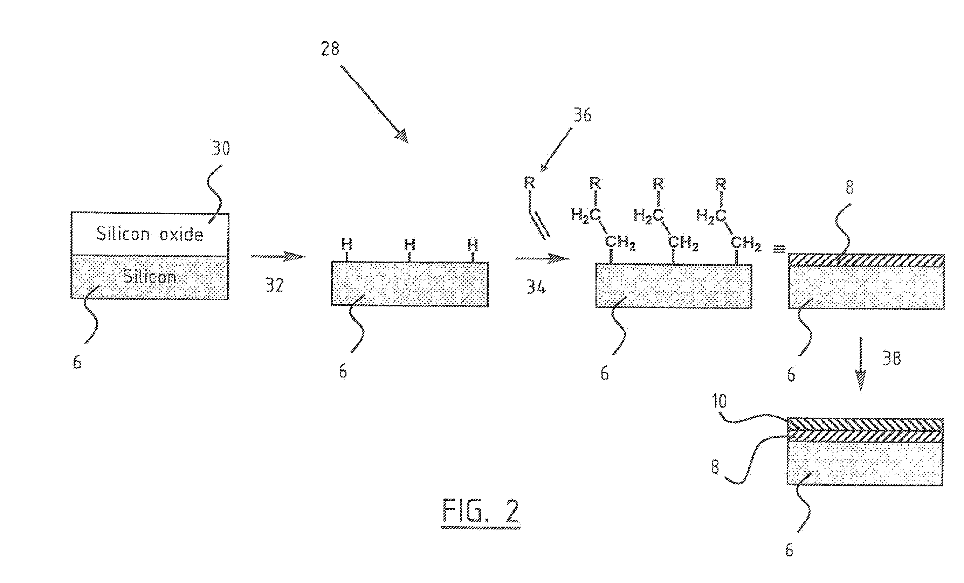

The production. 28 (figure 2) of a working electrode 4 starts with the silicon substrate 6. On top of substrate 6 there is provided a thin layer of a silicon oxide 30. By etching 32 this silicon oxide layer 30 is removed and a hydrogen-terminated silicon surface is performed. By hydrosilylating 34, 36 a Si-C linked organic monolayer 8 is formed. On top of monolayer 8 the affinity layer 10 is deposited 38.

Mono-layer 8 is connected to substrate 6 with a double bond (alkenes) or triple bond (alkynes). Mono-layer 8 can be functionalised with functional groups R (figure 2) or R' (not shown) respectively.

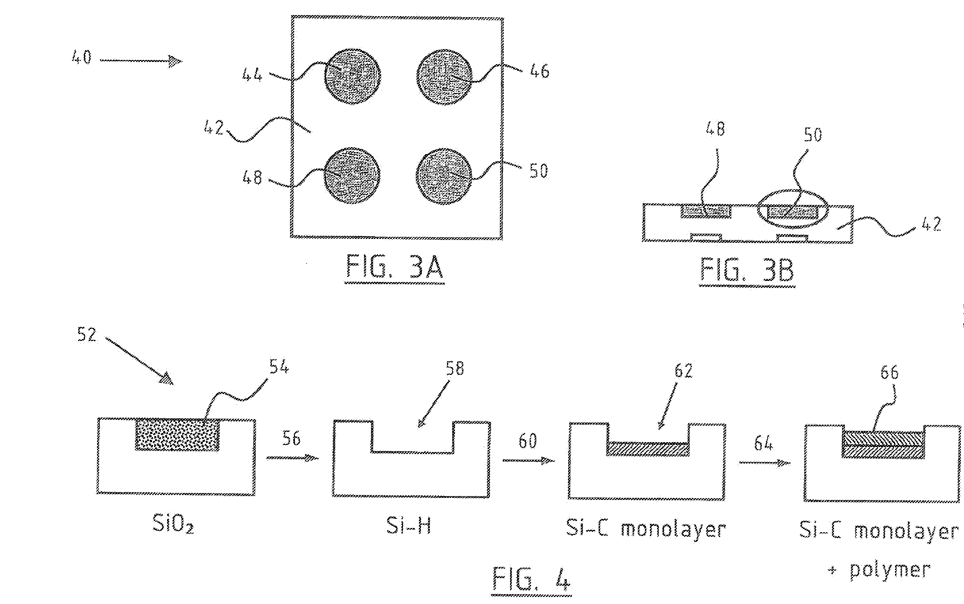

Multi-layer surface 40 (figure 3A and B) comprises base material 42 wherein spots 44, 46, 48, 50 are provided. Spots 44, 46, 48, 50 are made of different material, with different electric properties. The spots 44, 46, 48, 50 are provided by process 52 wherein the SiO₂ layer 54 is removed from the base material 42 by etching operation 56, This results in a hydrogen-terminate silicon 58. Surface 58 is provided with ain organic monolayer SiC-linked in step 60. In depositing step 64 affinity layer 66 is added. Molecular imprinted polymers can be used as an affinity layer. These layers are produced by process 68. Base material is provided with polymer material 72 and particles 74. After the depositing step 76 an affinity layer results. Particles 74 exchange with particles 80 in steps 78, 80.

In one of the embodiments the affinity layer is made of a micro-porous polypropylene membrane (accurel). The pores are filled with specific extraction fluids that interact with contaminants in the fluid. Changes in capacitance changes can be measured and indicate the presence of specific components and/or concentrations thereof in the fluid, Alternatively, polymer coatings filled with a solvent can be used as affinity layer. The adsorption properties of the coating can be manipulated by varying the composition of the solvent. A change in properties of such a filled polymer in time can be (automatically) corrected. Furthermore, the stability of polymer coatings during the filling can be manipulated by applying specific cross linking agents and chain transfer agents during the production of the polymer coating.

The electrode according to the invention can be used as a micro-reactor that can be a microbial fuel cell (MFC). By using the electrode as an anode or as a cathode, the fuel cell can be used as a bio toxicity sensor. The coating on the electrodes is selected depending on the specific toxic components for which the fuel cell should be sensitive. Another application of the electrodes according to the present invention is the use as a pro-active scaling sensor. In a supersaturated solution, nucleation is initiated by exposing the solution to ultrasonic vibrations. Using the electrodes according to the present invention enables measurement of extremely low concentration of agents and scale inhibitors. Using the sensor enables a. dozing strategy the scale inhibitor, thereby improving the efficiency of the process, The same applies in case an electric activator is used in stead of an ultrasonic activator.

The electrodes according to the present invention can also act as a so-called coax sensor wherein water acts as di-electricum between two concentric insulated conductors, These conductor are part of an antenna system and act as electric suction filter. The frequency of this filter changes as a response to a change in electric properties of the water due to contaminants therein. A frequency change can be measured using radio frequent alternating voltage technology. The use of electrodes according to the present invention results in a sensor that is more selective and sensitive. This improved sensitivity is realised by the adsorption characteristics of components in the water in relation to the coating. Furthermore, this selectivity can be manipulated by providing a potential difference between the two concentric insulated conductors.

Next, without hereby imposing any limitation on the scope of the present invention, a number of embodiments of the present invention will now be elucidated of a sensor-system for detecting contaminants making use of photovoltaic means and an "electrode" comprising a functionalized surface. A sensor-system 84 measures contaminations in tube 86 whereon and/or wherein a surface area 88 is provided that is functionalized. Source 90 transmits light 94 through tube 86 towards surface 88 that is (partly) received by receiver 92. This enables measurement of contaminations in a fluid present in tube 86 in a manner described before. Further embodiments, options and/or alternatives will be discussed next.

In a first embodiment a functionalised surface is placed in the light path between a light source and a PV cell. The functionalized surface is to the light produced by the light source. A non-limitative example of such a surface is glass or quartz to which a preferably thin coating is applied. Thin is understood in this case to mean a coating which preferably has a thickness in the range of 0.1 nanometre to 100 micrometres. The properties of the coating are such that it preferably adsorbs components present in the liquid. If components present in the liquid adsorb to the surface, the transmission properties of the surface will change, with the result the PV cell detects a change in light intensity, this being measurable in the form of a change in the current supplied by the PV cell. The light source and/or the PV cell can be situated outside the liquid as well as in the liquid. The functionalized surface to which adsorption takes place is situated in the liquid.

In a second embodiment a. PV cell is applied which is situated in the water. Applied to the PV cell is a coating which prevents corrosion of the PV cell. This coating also serves as surface to which contaminants adsorb.

In a third embodiment a solar cell is applied which is situated in the water and is specially designed for the electrolysis of water. The solar cell is equipped with a very thin coating which prevents corrosion of the solar cell but which guides electrons through the small thickness of the coating. Through the choice of the band gap of the solar cell the potential on the coating can be adjusted. It is hereby possible to apply the solar cell for capacitive deionization. Depending on the potential (the band gap applied) and the composition of the water, different types of contaminant in the water will adsorb to the coating. It will be apparent to the skilled person that this creates unprecedented possibilities for making the sensor sensitive and specific for detection of a variety of compounds, all the more so because not only the nature of the coating but also the potential can now be used as control parameter to bring about adsorption of components present in the water. It is finally noted that, at a band gap which is sufficiently large, not only does capacitive deionization occur but also electrolysis. This has the result that organic molecules present in the water will decompose as a result of, among others, OH, O and Cl radicals. The decomposition products will or will not adsorb well to the functionalized surface and will or will not absorb light well. It will be apparent to the skilled person that use can be made of these phenomena to control the selectivity of a sensor according to the present invention.

In a fourth embodiment a sensor according to any of the foregoing embodiments is combined with or partially replaced by a sensor which detects light on the basis of a photodiode, phototransistor, light-dependent resistor (LDR), photomultiplier on the basis of vacuum technology, photomultiplier on the basis of solid-state technology, antenna and/or amplifier for radio waves and/or alternating voltages in the range of 0.01 Hz to 1000 GHz, or by a so-called ChemFET (Field Effect Transistor with a Chemically modified gate electrode surface). It is noted that a ChemFET has the drawback relative to the present invention that an electrical circuit, including an external power supply with electrical energy, must be constructed around the FET. In the case a solar cell is applied according to the principle of the present invention, the measuring system can be realized more simply, more cheaply and without electric power supply. It can be advantageous to apply systems wherein solar cells and FETs and/or ChemFETs are combined. Such systems expressly form part of the present invention.

In a fifth, embodiment a sensor according to any of the foregoing embodiments is used to detect the light produced by or chemoluminescent organisms. It is know to the skilled person that the amount of light produced by these organisms can be used as a measure for the presence of toxic components in water. The functionalized surface of the PV cell or of the glass or quartz is preferably chosen such that light-emitting micro-organisms form a biofilm on this surface. A particular application of light-emitting micro-organisms is the arranging of a biofilm with these organisms on a solar cell surface, wherein the activity of the micro-organisms determines the potential of this surface. As a result the micro-organisms will begin to behave differently, this increasing the sensitivity of the sensor. It is noted that a solar cell with a biofilm of light micro-organisms on the surface forms a type of microbial fuel cell. Since recent developments in solar cell technology make it possible to produce solar cells with an adjustable small band gap, there are numerous possibilities for optimizing such a fuel cell.

In a sixth embodiment anodophilic and/or cathodophilic bacteria as applied, in a microbial fuel cell are arranged on a solar cell which has a configuration as described is any of the above embodiments. We then obtain a new type of microbial fuel cell. When light is now incident on the solar cell, the potential of the surface will change, It will be apparent to the skilled person that the amount of energy which the solar cell produces can in this way be combined with the electrical energy produced by the micro-organisms. In short, this means that water can be decomposed in very efficient manner since a solar cell-assisted microbial electrolysis cell has been created. Such a system can be applied as sensor, but also to produce hydrogen from wastewater and sunlight. It will be apparent to the skilled person that the system can also be applied to disinfect water.

In a seventh embodiment a sensor according to any of the above embodiments 1 to 6 is used to study and/or inhibit and/or enhance the metabolism of algae, Use can be made for this purpose of the fact that algae dispersed, in the water absorb light or that algae can adhere to a surface, whereby this surface absorbs light of a determined frequency spectrum.

In an eighth embodiment the surface of a quartz bottle or a PET bottle or other container is equipped on the inner side with a solar cell, or solar cells are arranged according to any of the above embodiments 1 to 7 in such a container. This provides the option of disinfecting and/or analyzing water in the sun and/or producing hydrogen art low cost and without corroding electrodes.

In a ninth embodiment a sensor according to any of the above embodiments 1 to 7 is applied in the pharmaceutical industry and/or the chemical industry and/or the biochemical industry and/or food industry to characterize intermediate products, end products and to determine quickly by means of fingerprinting whether a product meets the set requirements therefor.

In a tenth embodiment a number of sensors according to any of the above embodiments 1 to 9 is combined so as to thus obtain an array of sensors which together provide more information than the sum of the information provided by each of these sensors separately,

In an eleventh, embodiment at least one of the sensors described in embodiments 1 to 10 is combined with an optical sensor, wherein a measurement is made by means of Mach Zehnder interferometry of whether components have been adsorbed to a surface. It is known to the skilled person that a sensor operating according to the Mach Zehnder interferometry principle is based on a phase difference measurement of light transmitted through a surface to which components have been adsorbed and light which is transmitted through the same surface to which no components have been adsorbed. In the technique according to the present invention a difference measurement is desirable in a number of cases, wherein the energy production of a solar cell, wherein light is incident through a to which components have been adsorbed, is compared to the energy production of the same solar cell wherein light is incident through a surface to which no components have been adsorbed. It is however noted that, when the solar cell is applied, use can first be made of light with a frequency which is not absorbed by adsorbed components. This measurement is referred to as the zero measurement. Light is then transmitted through the same solar cell with a frequency at which absorption does occur by components adsorbed to the surface. By determining the quotient of the amplitude of the two signals a relative measure is obtained for the light absorption of the adsorbed components, This procedure can be repeated in automated manner for a wide frequency spectrum, whereby it is possible to map very precisely which substance our combination of substances has been adsorbed to the surface, without a difference measurement with multiple cells being necessary for this purpose. It will be apparent to the skilled person that the band gap of the solar cell determines to a significant extent the width of the frequency spectrum to which the solar cell is sensitive. For this reason a multi-array sensor wherein solar cells are applied with band gaps, in addition to the application with a solar cell, is a powerful aid for fingerprinting of water,

In a twelfth embodiment at least one of the sensors described in embodiments 1 to 11 is combined with a capacitive sensor which measures whether components have been adsorbed to a surface.

In a thirteenth embodiment at least one of the sensors described in embodiments 1 to 12 is combined with a sensor based on a microbial fuel cell.

In another embodiment an indirect measurement is performed. A water sample is purged with a gas such that the organic contaminations are transferred to the gas phase. The gas phase contacts the sensing layer and contaminations are transferred to the sensing or membrane layer. Experiments have shown that octane can be measured in the gas phase with a resolution of 2 ppm, for example.

The present invention is by no means limited to the above described preferred embodiments thereof. The rights sought are defined by following claims within the scope of which many modifications can be envisaged. For example, it is possible to combine the electrode according to the present invention in a sensing-system that also uses optical, mechanical and/or piezo electrical measurement. Furthermore, in case of an application involving adsorbed micro-organisms, these micro-organisms can be characterised using for example, interspectroscopy.

## Claims

1. Capacitance electrode for use in a sensor-system capable of capacitively sensing contaminants in a fluids the electrode comprising:
- a transducing element;
- an affinity layer that in use is in contact with a fluid to be measured, wherein the affinity layer comprises a multi-array surface; and
- an insulating layer separating the transducing element
and the affinity layer,
wherein the electrode is arranged such that in use contaminants in the fluid may interact with the affinity layer.

2. Capacitance electrode according to claim 1, wherein the multi-array surface comprises polymers and/or polymeric mixtures.

3. capacitance electrode according to claim 1 or 2, wherein the insulating layer comprises an organic mono-layer.

4. Capacitance electrode according to claim 1, 2 or 3, wherein the insulating layer comprises an alkene and/or alkyne that is provided with a functionalised component.

5. Capacitance electrode according to any of claims 1-4, wherein the affinity layer substantially comprises particles smaller than 1 pm, such that the layer substantially acts as a conductor when exposed to a high-frequent alternating current and acts as an insulator when exposed to a direct current.

6. Sensor-system for measuring contaminants in a fluid, comprising:
- a working electrode comprising a capacitance electrode according to any of claims 1-5;
- a counter electrode arranged such that in use fluid to be measured separates the working and counter electrode; and
- a potentiostat capacitively sensing the change in electric properties caused by adsorption of contaminants to the affinity layer.

7. Sensor-system according to claim 6, wherein at least the affinity layer of the working electrode is arranged in a cylindrical tube.

8. sensor-system according to claim 6, or 7, further comprising flow means capable to expose the sensor to a substantially continuous flow of fluid.

9. Sensor-system according to claim 6, 7 or 8, further comprising electrical means for providing a potential difference between the working and counter electrode.

10. Sensor-system according to any of claims 6-9, further comprising additional electrical means for providing an alternating electrical field between the working and counter electrode.

11. Sensor-system according to any of claims 6-10, wherein the counter electrode comprises an affinity layer.

12. Sensor-system for detecting contaminations in a fluid and/or for treating a fluid, comprising:
- a working element comprising an affinity layer that in use is in contact with a fluid to be measured, and wherein the working element is arranged such that in use contaminants in the fluid may interact with the affinity layer; and
- photovoltaic means.

13. Micro-reactor comprising a sensor-system according to any of claims 6-12.

14. Method for measuring contaminants in a fluid, in the pharmaceutical, chemical, medical and/or biochemical industry or applications comprising the steps of:
- providing a sensor-system according to any of claims 6-12; and
- capacitively or photovoltaic sensing the change in properties of the affinity layer due to adsorption of contaminants to the affinity layer.

15. Method for producing a capacitance electrode and/or a working element according to any of claims 1-5 and/or a sensor-system according to any of claims 6-12, comprising the steps of:
- etching a silicon surface;
- hydrosilylating the silicon surface to form a linked organic monolayer; and
- depositing the affinity layer,
preferably involving different polymers and/or polymeric mixtures for forming a multi-array affinity layer.
